(19) Europäisches Patentamt
European Patent Office
Office européen des brevets

(11) **EP 3 805 810 A1**

(12) **EUROPÄISCHE PATENTANMELDUNG**

(43) Veröffentlichungstag:
**14.04.2021 Patentblatt 2021/15**

(51) Int Cl.:
***G01T 1/29*** *(2006.01)*

(21) Anmeldenummer: **19201712.7**

(22) Anmeldetag: **07.10.2019**

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**
Benannte Validierungsstaaten:
**KH MA MD TN**

(71) Anmelder: **Siemens Healthcare GmbH**
**91052 Erlangen (DE)**

(72) Erfinder:
• **Tschentscher, Malte**
**90419 Nürnberg (DE)**
• **Kanter, Marcel**
**91054 Erlangen (DE)**

(54) **RÖNTGENBILDGEBUNGSVORRICHTUNG UND RÖNTGENBILDGEBUNGSVERFAHREN**

(57) Die Erfindung schafft ein Röntgen-Bildgebungsverfahren und eine Röntgen-Bildgebungsvorrichtung. Die Bildgebungsvorrichtung ist ausgebildet mit: einer Röntgenlichtquelle (10) zum Aussenden von Röntgenstrahlung (2);

einem Aufnahmebereich (30) für ein mit der Röntgenstrahlung (2) abzutastendes Objekt (1);

eine Umwandlungseinrichtung (40) zum räumlich aufgelösten Umwandeln von Röntgenstrahlung (2), welche den Aufnahmebereich (30) passiert hat, in sichtbares Licht;

einer Detektoreinrichtung (60), welche zum Ausgeben eines Detektorsignals (61) basierend auf durch die Detektoreinrichtung (60) erfasstem sichtbaren Licht aus der Umwandlungseinrichtung (40) ausgebildet ist;

einer Mustergeneratoreinrichtung (50), mittels welcher zeitversetzt eine Vielzahl von Licht-und-Schatten-Mustern, LSM, (51) durch Einwirken auf Röntgenstrahlung (2), welche den Aufnahmebereich (30) passiert hat und/oder auf von der Umwandlungseinrichtung (40) erzeugtes sichtbares Licht derart realisierbar ist, dass jedes Detektorsignal (61) basierend auf einem jeweiligen LSM (51) erzeugt wird; und

eine Auswerteeinrichtung (60), welche dazu ausgelegt ist, basierend auf den Detektorsignalen (61) sowie auf einer vorbestimmten Information über die LSM (51) das Abbild (5) des Objekts (1) zu generieren.

FIG 1

EP 3 805 810 A1

**Beschreibung**

Die vorliegende Erfindung betrifft eine Röntgenbildgebungsvorrichtung und ein Röntgenbildgebungsverfahren, insbesondere zum Durchführen einer Geisterbildgebung (engl.: "Ghostimaging").

In der konventionellen (Projektions-) Röntgentechnik ist die Bildqualität proportional zur eingesetzten Photonen-Flussdichte, welche wiederum mit einer entsprechenden Strahlenbelastung (Strahlendosis) des Objekts einhergeht. Eine möglichst weitgehende Reduktion der Strahlendosis bei Wahrung einer akzeptablen Bildqualität ist somit eine wichtige Herausforderung der medizinischen Röntgenbildgebung. Bei hochaufgelösten Aufnahmen besteht zudem bei der aktuellen Röntgenbildgebung eine Herausforderung durch Bildartefakte, welche anspruchsvolle Anforderungen hinsichtlich der Homogenität des Strahlengangs aufstellen.

Eine Möglichkeit zur Reduzierung der Strahlendosis auf ein Objekt bei der Bildgebung wird durch die so genannte Geisterbildgebung (engl. "ghost imaging", manchmal auch "coincidence imaging") bereitgestellt.

Geisterbildgebung oder Zwei-Photon-Bildgebung oder Korrelierte-Photonen-Bildgebung bezieht sich auf ein Bildgebungsverfahren basierend auf der Korrelation zweier elektromagnetischer Strahlenpfade. Üblicherweise befindet sich in einem ersten der zwei Strahlenpfade ein Objekt, für das eine Bildgebung durchgeführt werden soll (d.h. welches gescannt werden soll). Hinter dem Objekt ist in dem ersten Strahlenpfad ein Ein-Pixel-Detektor (engl.: "bucket detector") angeordnet, der auf ihn einfallende Lichtstrahlen detektieren kann. In einem zweiten Strahlenpfad befindet sich ein hochauflösender Detektor, zum Beispiel eine Kamera mit einem Raster von Detektorpixeln, aber kein Objekt. Der Ein-Pixel-Detektor und der hochauflösende Detektor haben üblicherweise dieselbe effektive Detektorfläche.

Elektromagnetische Strahlen (oder, kurz: Lichtstrahlen, wobei diese nicht im sichtbaren Frequenzspektrum liegen müssen) werden dann derart geleitet, dass sie, falls kein Objekt vorhanden wäre, gleichzeitig auf dieselben Positionen jeweils auf dem Ein-Pixel-Detektor und dem hochauflösenden Detektor einfallen würden, d.h. beide gleichzeitig auf die obere linke Ecke usw. Mit den Lichtstrahlen wird die effektive Detektorfläche abgefahren (gescannt), d.h. die Lichtstrahlen werden so geleitet, dass sie nach und nach auf diese gesamte effektive Detektorfläche einfallen.

Eine Analyseeinrichtung erfasst es, wenn eines der Detektorpixel des hochauflösenden Detektors einen Lichteinfall gleichzeitig detektiert wenn auch der Ein-Pixel-Dektor einen Lichteinfall detektiert.

Wenn nun ein Objekt in dem ersten Strahlenpfad angeordnet wird, fahren die Lichtstrahlen in dem zweiten Strahlenpfad weiterhin nach und nach die effektive Detektorfläche ab. In dem ersten Strahlenpfad hingegen werden die Lichtstrahlen bisweilen von dem Objekt absorbiert werden und daher nicht auf den Ein-Pixel-Detektor auftreffen. Die Analyseeinrichtung erkannt dann, dass keine simultane Detektion stattgefunden hat, und durch das registrierende Pixel des hochauflösenden Detektors ist gleichzeitig klar, an welcher Position in dem ersten Strahlenpfad dies stattgefunden hat. Somit kann mittels des hochauflösenden Detektors in dem zweiten Strahlenpfad eine akkurate Silhouette des Objekts erzeugt werden, ohne dass dieser Lichtstrahlen detektiert, die mit dem Objekt interagiert haben.

Diese Technik hat eine Vielzahl von Anwendungsformen und erhöht insbesondere die Designfreiheit, da beispielsweise in den verschiedenen Strahlengängen verschiedene Arten von Lichtstrahlen verwendet werden können, solange deren räumlicher Gleichlauf, d.h. das korrelierte räumliche Abtasten der effektiven Detektorfläche, gegeben ist. Beispielsweise können in dem ersten Strahlenpfad verhältnismäßig schwache Lichtstrahlen (d.h. mit geringer Leistung bzw. geringem Photonenfluss) verwendet werden, die der Einzel-Pixel-Detektor erfassen kann, während in dem zweiten Strahlenpfad verhältnismäßig starke Lichtstrahlen verwendet werden können. Somit kann ein lichtempfindliches Objekt geschont werden, und im zweiten Strahlenpfad kann dafür ein weniger empfindlicher und somit üblicherweise kostengünstigerer Detektor verwendet werden. Bei der Röntgenbildgebung können somit im ersten Strahlenpfad Röntgenstrahlung mit vergleichsweise niedriger Photonen-Flussdichte eingesetzt werden.

Ein Nachteil bei diesen Verfahren ist es, dass die hochenergetische Röntgenstrahlung üblicherweise die Mustergeneration erschwert, und Strahlenoptiken zur separaten Detektion der Intensitätsverteilungen für die Licht- und Schattenmuster für gewöhnlich nicht zur Verfügung stehen.

Eine Weiterentwicklung der Geisterbildgebung besteht darin, dass nur ein einziger Strahlenpfad verwendet wird, und dass das bildgebende Licht (z.B. Röntgenstrahlung) stattdessen von der Lichtquelle in Form von verschiedenen Licht- und Schattenmustern ausgesendet wird, oder vor dem Auftreffen auf das Objekt durch einen Mustergenerator in solche Licht- und Schattenmuster umgewandelt wird. Hierzu wird zunächst für jedes Licht- und Schattenmuster i mittels eines räumlich hochauflösenden Detektors eine Intensitätsverteilung $I_i$ erfasst. Diese Intensitätsverteilung $I_i$ können in einem Speicher hinterlegt werden.

Für das Abtasten eines Objekts wird das Objekt sodann vor einem niedrigauflösenden Detektor, z.B. einen Ein-Pixel-Detektor ("bucket detector") angeordnet und mit den einzelnen Licht- und Schattenmustern i beleuchtet, wobei der niedrigauflösende Detektor jeweils die zugehörige Intensität $S_i$ erfasst.

Aus der räumlich 2-dimensional aufgelösten Intensitätsverteilung $I_i(x, y)$ kann unter Verwendung der jeweils bei/mit diesem Licht- und Schattenmuster aufgenommenen Intensität $S_i$ durch Ausnutzen der Korrelation das Geisterbild $G(x,y)$ berechnet werden zu:

$$G(x,y) = \langle SI(x,y) \rangle - \langle S \rangle \langle I(x,y) \rangle \approx \frac{1}{N}\sum_{i=1}^{N} S_i I_i(x,y) - \frac{1}{N^2}\sum_{i=1}^{N} S_i \sum_{i=1}^{N} I_i(x,y),$$

wobei über die Anzahl der Licht- und Schattenmuster von i=1..N summiert wird, und wobei S die aufintegrierte detektierte Intensität beschreibt.

Eine Anwendung dieses Verfahrens, welches auch als Computer-Geisterbildgebung (engl. "computational ghost imaging") bezeichnet wird, ist beispielsweise in der wissenschaftlichen Veröffentlichung von Zhang, He, et al.: "Tabletop x-ray ghost imaging with ultra-low radiation", Optica, Vol 5, No. 4, April 2018, p. 374-377, https://doi.org/10.1364/OPTICA.5.000374 beschrieben.

Auch bei diesen Verfahren bestehen jedoch große Herausforderungen für das Erzeugen von verlässlichen Strahlenoptiken für die hochenergetische Röntgenstrahlung, für das Umschalten zwischen einzelnen Licht-und-Schatten-Mustern und für die Reproduzierbarkeit von Ergebnissen.

Es ist daher eine Aufgabe der vorliegenden Erfindung, ein verbessertes Bildgebungsvorrichtung und ein verbessertes Bildgebungsverfahren bereitzustellen, welche insbesondere die o.g. Herausforderungen bewältigen.

Diese Aufgabe wird durch die unabhängigen Patentansprüche gelöst, wobei die abhängigen Patentansprüche vorteilhafte Weiterbildungen, Ausführungsformen und Varianten beschreiben.

Die Erfindung stellt somit zum einen eine Bildgebungsvorrichtung zum Erstellen eines Abbilds eines Objekts durch Bestrahlen des Objekts mit Röntgenstrahlung, bereit, umfassend:

einer Röntgenlichtquelle zum Aussenden von Röntgenstrahlung;
einem Aufnahmebereich für ein mit der Röntgenstrahlung abzutastendes Objekt;
eine Umwandlungseinrichtung zum räumlich aufgelösten Umwandeln von Röntgenstrahlung, welche den Aufnahmebereich passiert hat, in sichtbares Licht;
einer Detektoreinrichtung, welche zum Ausgeben eines Detektorsignals basierend auf durch die Detektoreinrichtung erfasstem sichtbaren Licht aus der Umwandlungseinrichtung ausgebildet ist;
einer Mustergeneratoreinrichtung, mittels welcher zeitversetzt eine Vielzahl von Licht-und-Schatten-Mustern, LSM, durch Einwirken auf Röntgenstrahlung, welche den Aufnahmebereich passiert hat und/oder auf von der Umwandlungseinrichtung erzeugtes sichtbares Licht derart realisierbar ist, dass jedes Detektorsignal basierend auf einem jeweiligen Licht-und-Schatten-Muster erzeugt wird; und
eine Auswerteeinrichtung, welche dazu ausgelegt ist, basierend auf den Detektorsignalen sowie auf einer vorbestimmten Information über die LSM ein Abbild des Objekts zu generieren.

Eine grundlegende Idee der vorliegenden Erfindung ist es somit, bekannte Computer-Geisterbildgebungsverfahren so abzuwandeln, dass Licht-und-Schatten-Muster, LSM, nicht quellennah sondern detektornah realisiert werden. Auf diese Weise wird eine quellenunabhängige Röntgen-Geisterbildgebung verwirklicht.

Die Umwandlungseinrichtung kann insbesondere einen Szintillator umfassen oder aus einem Szintillator bestehen. Unter einem "räumlich aufgelösten Umwandeln von Röntgenstrahlung" ist insbesondere zu verstehen, dass die aus dem Aufnahmebereich empfangene Röntgenstrahlung, welche an dem Objekt in dem Aufnahmebereich somit vorbei geleitet wurde, an der Umwandlungseinrichtung so umgewandelt wird, dass die räumliche Information der (teilweise an dem Objekt absorbierten) Röntgenstrahlung dadurch nicht verloren geht.

Die Mustergeneratoreinrichtung kann dabei gemäß einer Vielzahl von Varianten realisiert sein, welche im Folgenden näher erläutert werden. Bevorzugt interagiert die Mustergeneratoreinrichtung ausschließlich mit sichtbarem Licht, insbesondere von der Umwandlungseinrichtung erzeugtem Licht, um die Herausforderungen im Umgang mit hochenergetischer Röntgenstrahlung zu umgehen.

Vorteilhaft ist die Mustergeneratoreinrichtung dazu ausgelegt, sichtbarem Licht die Licht-und-Schatten-Muster aufzuprägen, sodass aufwändige Strahlenoptiken zur Mustergeneration in/an Röntgenstrahlung entfallen können. Die Mustergeneration durch die Mustergeneratoreinrichtung kann beispielsweise durch Übersteuern (Pixel weiß machen, egal ob sie ursprünglich schwarz, grau oder weiß waren) und/oder durch Abschatten (Pixel schwarz machen, egal ob sie ursprünglich schwarz, grau oder weiß waren) erfolgen.

Dass jedes Detektorsignal basierend auf einem jeweiligen Licht-und-Schatten-Muster erzeugt wird, wird beispielsweise derart bewirkt, dass durch das Licht-und-Schatten-Muster, LSM, das auf den Detektorsignal auftreffende sichtbare Licht durch Übersteuern und/oder Abschatten modifiziert ist.

Die Mustergeneratoreinrichtung ist weiterhin vorteilhaft derart ausgebildet, dass die Licht-und-Schatten-Muster, die zeitversetzt (d.h. nacheinander) realisiert werden, linear unabhängig sind. Auf diese Weise trägt die Information aus jedem Abtastvorgang neue räumliche Informationen für die Abbildung des Objekts bei. Weiterhin kann die Mustergeneratoreinrichtung dazu ausgebildet sein, die Licht-und-Schatten-Muster, LSM, diskret zu realisieren, oder dazu ausgebildet sein, die Licht-und-Schatten-Muster, LSM, kontinuierlich zu realisieren.

Die vorbestimmte Information über die Licht-und-Schatten-Muster, LSM, ist vorzugsweise in einem nicht-flüchtigen, computerlesbaren Datenspeicher der Auswerteeinrichtung gespeichert. Die vorbestimmte Information über die Licht-und-Schatten-Muster, LSM, umfasst bevorzugt eine vorbestimmte Information über jedes einzelne Licht-und-Schatten-Muster, LSM, was insbesondere vorteilhaft ist, wenn zeitgenau bekannt ist, welches Licht-und-Schatten-Muster, LSM, gerade durch die Mustergeneratoreinrichtung realisiert wurde, während die Detektoreinrichtung sichtbares Licht erfasst.

Es wird darauf hingewiesen, dass vorliegend die Lichtgeschwindigkeit bei allen Betrachtungen ignoriert wird, und somit Ereignisse, die nur aufgrund der Lichtgeschwindigkeit voneinander versetzt stattfinden, als gleichzeitig bezeichnet werden.

Bei der vorbestimmten Information über das jeweilige Licht-und-Schatten-Muster, LSM, handelt es sich vorzugsweise um eine jeweilige Intensitätsverteilung $I_i(x,y)$, welche räumlich aufgelöst für jedes Licht-und-Schatten-Muster, LSM, mittels eines räumlich auflösenden Detektors ermittelt und gespeichert wurde, etwa wie im Vorangehenden bereits erläutert wurde.

Alternativ kann die vorbestimmte Information auch lediglich die räumliche Gestaltung des jeweiligen Licht- und Schattenmusters selbst sein, insbesondere wenn es sich bei den Licht-und Schattenmustern um bekannte und klar definierte Muster handelt, deren Lichtintensitäten bei Abwesenheit eines Objekts ohne Messung berechnet werden können.

Weiter alternativ kann vorgesehen sein, dass von der Detektoreinrichtung empfangene Lichtintensitäten (beispielsweise schon durch die Detektoreinrichtung selbst) aufintegriert (oder aufsummiert) werden. In diesem Fall kann es auch genügen, wenn eine vorbestimmte Information über die Gesamtheit der Licht-und-Schatten-Muster, LSM, welche die aufintegrierten/aufsummierten Lichtintensitäten bewirkt haben, bekannt ist.

Das Detektorsignal kann insbesondere eine gemessene Lichtintensität (etwa im Fall eines Ein-Pixel-Detektors als Detektoreinrichtung) oder eine Mehrzahl von gemessenen Lichtintensitäten (im Falle eines niedrigauflösenden Detektors mit wenigen Detektorpixeln als Detektoreinrichtung) umfassen oder beschreiben.

Die beschriebene Bildgebungsvorrichtung hat beispielsweise den Vorteil, dass durch die Verwendung einer von der Röntgenlichtquelle unabhängigen Mustergeneratoreinrichtung auch die Anforderungen an die Röntgenlichtquelle hinsichtlich der Reproduzierbarkeit sinken. Zudem wird es möglich, die Licht-und-Schatten-Muster, LSM, an die gewünschte Auflösung von Ortsfrequenzen anzupassen, was der Bildgebung einen weiteren Freiheitsgrad verschafft.

In einigen Ausführungsformen, Weiterbildungen oder Varianten von Ausführungsformen ist die Mustergeneratoreinrichtung zwischen der Umwandlungseinrichtung und der Detektoreinrichtung angeordnet und erzeugt die Licht-und-Schatten-Muster durch partielles Abschatten und/oder Übersteuern des sichtbaren Lichts. Auf diese Weise steht eine Vielzahl von bekannten Mustergeneratoreinrichtungen zur Verfügung, z.B. grating light valves und dergleichen zur Verfügung, was eine hohe Designfreiheit mit sich bringt.

In einigen Ausführungsformen, Weiterbildungen oder Varianten von Ausführungsforme sind die Mustergeneratoreinrichtung und die Umwandlungseinrichtung derart ineinander integriert, dass die Umwandlungseinrichtung nur Röntgenstrahlung, welche auf gemäß dem jeweiligen LSM bestimmte Bereiche der Umwandlungseinrichtung auftrifft, in sichtbares Licht umwandelt. Auf diese Weise sind die Licht-und-Schatten-Muster bereits bei der Erzeugung des sichtbaren Lichts auf das sichtbare Licht aufgeprägt. Zudem kann eine solche Anordnung besonders platzsparend realisiert werden.

Die Mustergeneratoreinrichtung und die Umwandlungseinrichtung können auch auf andere Art und Weise ineinander integriert sein. Beispielsweise kann eine Speicherfolie vorgesehen sein, welche Anregungen durch Röntgenstrahlung speichert und durch sichtbares Licht räumlich aufgelöst auslesbar ist. Ein Licht-und-Schatten-Muster, LSM, kann somit durch gezieltes räumliches Auslesen der Speicherfolie realisiert werden.

In einigen Ausführungsformen, Weiterbildungen oder Varianten von Ausführungsformen sind die Mustergeneratoreinrichtung und die Detektoreinrichtung derart ineinander integriert, dass das Licht-und-Schatten-Muster direkt auf der Detektoreinrichtung erzeugt wird. Auch eine solche Anordnung kann besonders platzsparend realisiert werden.

In einigen Ausführungsformen, Weiterbildungen oder Varianten von Ausführungsformen weist die Detektoreinrichtung eine Auflösung von größer gleich fünf und kleiner gleich einhundert Detektorpixeln auf, bevorzugt von größer gleich fünf und kleiner gleich fünfzig Detektorpixeln, besonders bevorzugt von größer gleich fünf und kleiner gleich fünfundzwanzig Detektorpixeln. Solche Sensoren sind mit verhältnisweise geringem technischen Aufwand realisierbar und können entsprechend - bei insgesamt gleichbleibendem technischen Aufwand - mit höherer Sensitivität ausgebildet werden. Dies wiederum ermöglicht es, bei der Abtastung Röntgenstrahlung mit geringerer Sensitivität zu verwenden, wodurch das abzutastende Objekt (das heißt das zu röntgende Objekt) vorteilhaft mit einer geringeren Strahlendosis belastet wird.

In einigen Ausführungsformen, Weiterbildungen oder Varianten von Ausführungsformen werden mehrere Detektorpixel zur Auswertung zu einer Ausleseeinheit zusammengefasst ("pixel-binning"). Auf diese Weise kann die Sensitivität eines bestehenden Detektors weiter erhöht werden. Da die räumliche Information im Wesentlichen in den Licht-und-Schatten-Mustern, LSM, sowie in den für diese bekannten Intensitätsverteilungen $I_i$ codiert ist, können bei der vorliegenden Erfindung Abwägungen zwischen Pixelanzahl der Detektoreinrichtung einerseits und Sensitivität der Detektoreinrichtung andererseits durchgeführt werden, ohne dadurch notwendigerweise Abstriche bei der räumlichen Auflösung machen zu müssen.

In einigen Ausführungsformen, Weiterbildungen oder Varianten von Ausführungsformen ist die Detektoreinrichtung als ein Ein-Detektorpixel-Detektor (engl. "bucket detector") ausgebildet. Bei diesem Extremfall liegt eine hohe Sensitivität bei gleichzeitig geringem technischem Aufwand der Detektoreinrichtung vor, andererseits muss eine vergleichsweise hohe Anzahl von Licht-und-Schatten-Mustern, LSM, verwendet werden, um genügend Informationen zur Generierung des Abbilds (d.h. zur Rekonstruktion) des Objekts zu erhalten. Eine höhere Anzahl von Licht-und-Schatten-Mustern, LSM, bedeutet ceteris paribus, insbesondere bei gleichbleibender Bestrahlungszeit pro Licht-und-Schatten-Muster, LSM, eine längere Bestrahlungszeit und somit eine höhere Strahlenbelastung des Objekts. Je nach gewünschter Anwendung und Sensibilität des Objekts gegenüber Röntgenstrahlung kann somit auch die Detektorpixelanzahl der Detektoreinrichtung individuell angepasst werden. Die Erfindung bietet somit einem Entwickler eine Vielzahl von Gestaltungsmöglichkeiten und Stellschrauben, um für jede Anwendung die optimale Konfiguration einrichten zu können.

In einigen Ausführungsformen, Weiterbildungen oder Varianten von Ausführungsformen ist eine Auflösung der Licht-und-Schatten-Muster, LSM, größer als eine Auflösung der Detektoreinrichtung. Auch dies ermöglicht es, die räumlichen Informationen im Wesentlichen in den Licht-und-Schatten-Mustern, LSM, und den zugehörigen Intensitätsverteilungen, zu codieren, wodurch die Auflösung der Detektoreinrichtung kostensparend gering gehalten werden kann.

In einigen Ausführungsformen, Weiterbildungen oder Varianten von Ausführungsformen ist die Bildgebungsvorrichtung so ausgebildet, dass die Gesamtzahl der Licht- und Schattenmuster, LSM, die nacheinander durchlaufen werden für eine einzelne Bildgebung, geringer als die Gesamtzahl der Pixel der Intensitätsverteilungen I(x,y) für die Licht-und-Schatten-Muster, LSM, oder zumindest geringer als die Gesamtzahl der Pixel der Intensitätsverteilung I(x,y) für die Licht-und-Schatten-Muster, LSM, multipliziert mit der Anzahl der Detektorpixel der Detektoreinrichtung. Auf diese Weise kann gegenüber einem konventionellen Abtasten des Objekts und Verwendung eines hochauflösenden Detektors eine zu übertragende Datenmenge verringert werden, da für jedes Licht-und-Schatten-Muster, LSM, nur jeweils ein gemessener Intensitätswert pro Detektorpixel der Detektoreinrichtung an die Auswerteeinrichtung übertragen werden muss, wenn die zuvor gemessenen Intensitätsverteilungen I(x,y) der Auswerteeinrichtung bereits vorliegen. Datenspeicher- und/oder Datenübertragungskapazitäten der Detektoreinrichtung können somit geringer gewählt werden, was wiederum den technischen Aufwand und die Kosten der Bildgebungsvorrichtung verringern kann.

In einigen Ausführungsformen, Weiterbildungen oder Varianten von Ausführungsformen wird eine Zeitintegration oder Summierung der Detektorsignale in der Detektoreinrichtung durchgeführt. Auf diese Weise kann eine Datenmenge, welche von der Detektoreinrichtung beispielsweise an die Auswerteeinrichtung übertragen werden muss, verringert werden.

Die Bildgebungsvorrichtung kann auch derart ausgebildet und eingerichtet sein, dass eine kontinuierliche Beleuchtung des Objekts mit Röntgenstrahlung und kontinuierliche Musterwechsel der Licht-und-Schatten-Muster, LSM, durchgeführt werden, und die von der Detektoreinrichtung erfassten Lichtintensitäten integriert werden. Auf diese Weise wird die Problematik der nötigen schnellen Detektorauslesungen umgangen. Zudem werden durch die der vorliegenden Erfindung zugrunde liegende Korrelationsmethode, d.h. das Ausnutzen der Korrelation zwischen den bekannten Lichtintensitäten der Licht-und-Schatten-Muster und den gemessenen Lichtintensitäten nach Abtasten des Objekts, automatisch auftretende Systemartefakte eliminiert.

Somit werden beispielsweise Inhomogenitäten, bedingt durch Systemunreinheiten im Strahlengang, verringert oder sogar eliminiert.

Die Erfindung stellt weiterhin ein Bildgebungsverfahren zum Erstellen eines Abbilds eines Objekts durch Bestrahlen des Objekts mit Röntgenstrahlung bereit, umfassend die Schritte:

Anordnen eines abzutastenden Objekts in einem Aufnahmebereich;
Aussenden von Röntgenstrahlung durch eine Röntgenlichtquelle in den Aufnahmebereich zum Beleuchten des abzutastenden Objekts;
räumlich aufgelöstes Umwandeln von Röntgenstrahlung, welche den Aufnahmebereich passiert hat, in sichtbares Licht;
Erzeugen von Detektorsignalen (z.B. je eines Detektorsignals pro Licht-und-Schatten-Muster, LSM) basierend auf durch eine Detektoreinrichtung erfasstem sichtbaren Licht;
Zeitversetztes Erzeugen einer Vielzahl von Licht-und-Schatten-Mustern, LSM, durch Einwirken auf Röntgenstrahlung, welche den Aufnahmebereich passiert hat und/oder auf von der Umwandlungseinrichtung erzeugtes sichtbares Licht derart, dass jedes Detektorsignal basierend auf einem jeweiligen Licht-und-Schatten-Muster erzeugt wird; und
Generieren, basierend auf den Detektorsignalen sowie auf einer vorbestimmten Information über die LSM, eines Abbilds des Objekts.

Die obigen Ausgestaltungen und Weiterbildungen lassen sich, sofern sinnvoll, beliebig miteinander kombinieren. Weitere mögliche Ausgestaltungen, Weiterbildungen und Implementierungen der Erfindung umfassen auch nicht explizit genannte Kombinationen von zuvor oder im Folgenden bezüglich der Ausführungsbeispiele beschriebenen Merkmale der Erfindung. Insbesondere wird dabei der Fachmann auch Einzelaspekte als Verbesserungen oder Ergänzungen zu der jeweiligen Grundform der vorliegenden Erfindung hinzufügen.

Weitere vorteilhafte Weiterbildung und Ausführungsformen werden im Folgenden anhand der Figuren und der zugehörigen Figurenbeschreibung erläutert werden.

Kurze Inhaltsangabe der Figuren

Die vorliegende Erfindung wird nachfolgend anhand der in den schematischen Figuren angegebenen Ausführungsbeispiele näher erläutert. Es zeigen dabei:

Fig. 1 ein schematisches Blockdiagramm zum Erläutern einer Bildgebungsvorrichtung gemäß einer Ausführungsform der vorliegenden Erfindung; und

Fig. 2 ein schematisches Flussdiagramm zum Erläutern eines Bildgebungsverfahrens gemäß einer weiteren Ausführungsform der vorliegenden Erfindung.

Die beiliegenden Figuren sollen ein weiteres Verständnis der Ausführungsformen der Erfindung vermitteln. Sie veranschaulichen Ausführungsformen und dienen im Zusammenhang mit der Beschreibung der Erklärung von Prinzipien und Konzepten der Erfindung. Die Elemente der Figuren sind nicht notwendigerweise maßstabsgetreu zueinander gezeigt. Richtungsangebende Terminologie wie etwa "oben", "unten", "links", "rechts", "über", "unter", "horizontal", "vertikal", "vorne", "hinten" und ähnliche Angaben werden lediglich zu erläuternden Zwecken verwendet und dienen nicht der Beschränkung der Allgemeinheit auf spezifische Ausgestaltungen wie in den Figuren gezeigt.

In den Figuren der Zeichnung sind gleiche, funktionsgleiche und gleich wirkende Elemente, Merkmale und Komponenten - sofern nichts anderes ausgeführt ist - jeweils mit denselben Bezugszeichen versehen.

Die Bezeichnungen von Verfahrensschritten dient der besseren Übersichtlichkeit; deren Nummerierung kann zwar, muss aber nicht notwendigerweise eine zeitliche Reihenfolge implizieren, sofern nichts Gegenteiliges explizit ausgesagt oder implizit ersichtlich ist. Insbesondere können mehrere Verfahrensschritte auch teilweise oder vollständig überlappen, d.h. insbesondere auch gleichzeitig durchgeführt werden.

Beschreibung der Ausführungsbeispiele

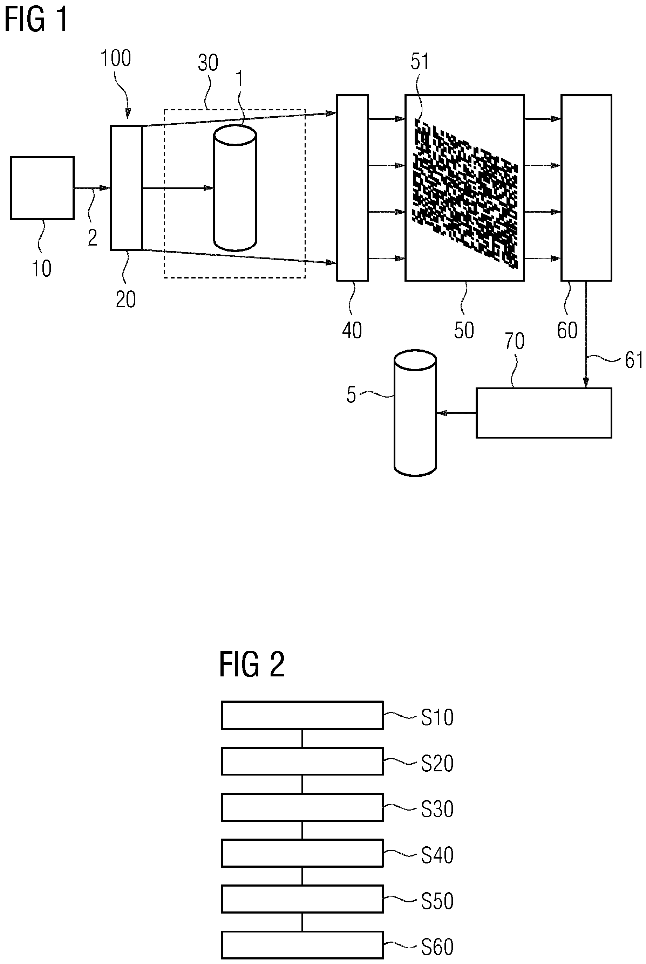

Fig. 1 zeigt ein schematisches Blockdiagramm zum Erläutern einer Bildgebungsvorrichtung 100 zum Erstellen eines Abbilds 5 eines Objekts 1 durch Bestrahlen des Objekts 1 mit Röntgenstrahlung 2 gemäß einer Ausführungsform der vorliegenden Erfindung.

Die Bildgebungsvorrichtung 100 umfasst eine Röntgenlichtquelle 10 zum Aussenden von Röntgenstrahlung 2 sowie einen einem Aufnahmebereich 30 für ein mit der Röntgenstrahlung 2 abzutastendes Objekt 1. Bei dem abzutastenden Objekt 1 kann es sich beispielsweise um ein Werkstück handeln, aber auch um ein lebendes Wesen wie z.B. einen menschlichen Patienten.

Der Aufnahmebereich 30 kann beispielsweise einen Tisch zum Auflegen des Objekts 1, eine verschließbare Kammer zum Aufnehmen des Objekts 1 oder dergleichen umfassen. Zwischen dem Aufnahmebereich 30 und der Röntgenlichtquelle 10 kann eine optische Einrichtung 20 vorgesehen sein, beispielsweise eine Apertur.

Im Strahlengang hinter dem Aufnahmebereich 30 ist eine Umwandlungseinrichtung 40 der Bildgebungsvorrichtung 100 angeordnet, welche dazu ausgebildet und eingerichtet ist, Röntgenstrahlung, welche den Aufnahmebereich 30 passiert hat, räumlich aufgelöst in sichtbares Licht umzuwandeln. Zu diesem Zeitpunkt wäre dem umgewandelten sichtbaren Licht somit noch die gesamte räumliche Information über das Objekt 1 entnehmbar. Wie im Vorangehenden beschrieben wurde, wäre hierfür aber eine starke Photonen-Flussdichte nötig, die für eine Vielzahl von Objekten 1 schädlich sein könnte. Bei der Umwandlungseinrichtung 40 kann es sich beispielsweise um einen Szintillator handeln.

Die Bildgebungsvorrichtung 100 umfasst weiterhin eine Detektoreinrichtung 60, welche zum Ausgeben eines Detektorsignals 61 basierend auf durch die Detektoreinheit 60 erfasstem sichtbaren Licht ausgebildet ist, wobei dieses sichtbare Licht vorzugsweise aus der Umwandlungseinrichtung 40 stammt. In anderen Varianten könnten zwischenzeitlich weitere Umwandlungen des erzeugten sichtbaren Lichts stattfinden. Die Bildgebungsvorrichtung 100 ist so ausgebildet, dass die Röntgenstrahlung 2, welche den Aufnahmebereich 30 passiert hat, so auf die Umwandlungseinrichtung 40 auftrifft, dass das von dieser (durch Umwandlung) erzeugte sichtbare Licht, nach Passieren der Mustergeneratoreinrichtung 50, auf die Detektoreinrichtung 60 auftrifft. Vorteilhaft erfolgt dies so, dass bei einem vollständig leeren Licht-und-Schatten-Muster, LSM, 51, welches alle Strahlung unverändert passieren lässt, auch die gesamte Röntgenstrahlung 2 aus dem Aufnahmebereich 30 auf die Detektoreinrichtung 60 auftrifft.

Wie im Vorangehenden bereits ausführlich erläutert wurde, kann die Bildgebungsvorrichtung 100 insbesondere einen Ein-Detektorpixel-Detektor umfassen (oder daraus bestehen), oder einen Detektor mit einer vergleichsweise geringen Auflösung aufweisen (oder daraus bestehen), wobei beträchtliche Vorteile realisiert werden können, wenn die Auflösung (d.h. Detektorpixelanzahl) der Detektoreinrichtung 60 geringer ist als eine Auflösung (d.h. Pixelanzahl) der Licht-und-Schatten-Muster, LSM, 51. Weiterhin kann auch ein pixel-binning angewendet werden, um die Sensitivität der Detektoreinrichtung 60 zu erhöhen und somit die nötige Belichtungszeit (oder Bestrahlungszeit) des Objekts 1 mit Röntgenstrahlung 2 pro Licht-und-Schatten-Muster, LSM, 51, zu verringern.

Weiterhin umfasst die Bildgebungsvorrichtung 100 eine Mustergeneratoreinrichtung 50, mittels welcher zeitversetzt eine Vielzahl von Licht-und-Schatten-Mustern, LSM, 51 durch Einwirken auf Röntgenstrahlung 2, welche den Aufnahmebereich 30 passiert hat und/oder durch Einwirken auf von der Umwandlungseinrichtung 40 erzeugtes sichtbares Licht derart realisierbar ist, dass jedes Detektorsignal 61 basierend auf einem jeweiligen Licht-und-Schatten-Muster, LSM, 51 erzeugt wird.

Vorteilhaft wirkt die Mustergeneratoreinrichtung 50 auf sichtbares Licht ein, sodass sie mit geringem Aufwand realisierbar ist. Die Mustergeneration durch die Mustergeneratoreinrichtung 50 kann beispielsweise durch Übersteuern (Pixel weiß machen, egal ob sie ursprünglich schwarz, grau oder weiß waren) und/oder durch Abschatten (Pixel schwarz machen, egal ob sie ursprünglich schwarz, grau oder weiß waren) erfolgen.

Die Mustergeneratoreinrichtung 50 ist weiterhin vorteilhaft derart ausgebildet, dass die Licht-und-Schatten-Muster, LSM, 51, die zeitversetzt (d.h. nacheinander) realisiert werden, linear unabhängig sind. Auf diese Weise trägt die Information aus jedem Abtastvorgang neue räumliche Informationen für die Abbildung des Objekts bei. Weiterhin kann die Mustergeneratoreinrichtung dazu ausgebildet sein, die Licht-und-Schatten-Muster, LSM, 51 diskret zu realisieren, oder dazu ausgebildet sein, die Licht-und-Schatten-Muster, LSM, 51 kontinuierlich zu realisieren.

Schließlich umfasst die Bildgebungsvorrichtung 100 eine Auswerteeinrichtung 70, welche dazu ausgelegt ist, basierend auf den Detektorsignalen 61 sowie auf einer vorbestimmten Information über die Licht-und-Schatten-Muster, LSM, 51 vorzugsweise über ein jeweiliges, zu einem Detektorsignal 61 gehöriges Licht-und-Schatten-Muster, LSM, 51 ein Abbild 5 des Objekts 1 zu generieren. Dies kann insbesondere mittels Computer-Geisterbildgebung, erfolgen, wie im Vorangehenden beschrieben wurde.

Insbesondere kann für jedes Licht-und-Schatten-Muster, LSM, 51, ein zugehörige Intensitätsverteilung I(x,y), aufgenommen mit einem hochauflösenden Detektor, vorbestimmt und in einem Datenspeicher der Auswerteeinrichtung 70 gespeichert sein. Unter Ausnutzung der Korrelation zwischen diesen Intensitätsverteilungen I(x,y), der jeweils durch die Detektoreinrichtung 60 gemessenen Intensität sowie der Information, welches Licht-und-Schatten-Muster, LSM, 51 welche Intensität an der Detektoreinrichtung 60 erzeugt hat, kann somit gemäß bekannten Algorithmen das Abbild 5 des Objekts 1 generiert werden.

In Fig. 1 wurde die Bildgebungsvorrichtung 100 exemplarisch derart dargestellt, dass Umwandlungseinrichtung 40, Mustergeneratoreinrichtung 50 und Detektoreinrichtung 60 jeweils als voneinander separate Blöcke abgebildet sind.

Wie im Vorangehenden jedoch erläutert wurde, kann es anwendungsabhängig vorteilhaft sein, mehrere oder sogar alle dieser Funktionsblöcke ineinander zu integrieren.

Beispielsweise können die Mustergeneratoreinrichtung 50 und die Umwandlungseinrichtung 40 derart ineinander integriert, dass die Umwandlungseinrichtung 40 nur Röntgenstrahlung 2, welche auf gemäß dem jeweiligen Licht-und-Schatten-Muster, LSM, 51 bestimmte Bereiche der Umwandlungseinrichtung 40 auftrifft, in sichtbares Licht umwandelt. Weiterhin kann eine Speicherfolie vorgesehen sein, welche Anregungen durch Röntgenstrahlung 2 speichert und durch sichtbares Licht räumlich aufgelöst auslesbar ist. Ein Licht-und-Schatten-Muster, LSM, 51 kann somit durch gezieltes räumliches Auslesen der Speicherfolie realisiert werden. Wieder alternativ können die Mustergeneratoreinrichtung 50 und die Detektoreinrichtung 60 derart ineinander integriert sein, dass das jeweilige Licht-und-Schatten-Muster, LSM, 51 direkt auf der Detektoreinrichtung 60 erzeugt wird.

Die in Fig. 1 schematisch dargestellte Bildgebungsvorrichtung 100 kann somit gemäß einer Vielzahl von im Vorangehenden beschriebenen Varianten und Optionen variiert werden.

Fig. 2 zeigt ein schematisches Flussdiagramm zum Erläutern eines Bildgebungsverfahrens zum Erstellen eines Abbilds 5 eines Objekts 1 durch Bestrahlen des Objekts 1 mit Röntgenstrahlung 2 gemäß einer weiteren Ausführungsform der vorliegenden Erfindung. Das Verfahren gemäß Fig. 2 ist insbesondere mittels der Bildgebungsvorrichtung 100 gemäß Fig. 1 durchführbar. Somit ist das Verfahren gemäß Fig. 2 auch gemäß allen bezüglich der Bildgebungsvorrichtung 100 beschriebenen Optionen, Varianten und Weiterbildungen anpassbar und umgekehrt.

In einem Schritt S10 wird ein abzutastendes Objekts 1 in einem Aufnahmebereich 30 angeordnet.

In einem Schritt S20 wird Röntgenstrahlung 2 durch eine Röntgenlichtquelle 10 zum Beleuchten des Aufnahmebereichs 30 und somit auch des Objekts 1 ausgesendet. Optional kann die Röntgenstrahlung vor Eintreten in den Aufnahmebereich 30 durch eine optische Einrichtung 20 geformt, z.B. fokussiert, werden.

In einem Schritt S30 wird Röntgenstrahlung 2, welche den Aufnahmebereich 30 passiert hat (d.h. nicht von dem Objekt 1 absorbiert oder völlig aus dem Strahlenpfad hinaus gestreut wurde), räumlich aufgelöst in sichtbares Licht umgewandelt, beispielsweise durch eine Umwandlungseinrichtung 40, wie im Vorangehenden ausführlich erläutert wurde.

In einem Schritt S40 wird zeitversetzt eine Vielzahl von Licht-und-Schatten-Mustern, LSM, 51 durch Einwirken auf Röntgenstrahlung 2, welche den Aufnahmebereich 30 passiert hat und/oder auf durch Umwandlung erzeugtes sichtbares Licht derart realisiert, dass ein in einem Schritt S50 durch eine Detektoreinrichtung 60 erzeugtes Detektorsignal 61 basierend auf sichtbarem Licht, das nach der Umwandlung auf die Detektoreinrichtung 60 auftritt sowie basierend auf einem jeweiligen Licht-und-Schatten-Muster, LSM 51, erzeugt wird. Wie beschrieben kann für jedes Licht-und-Schatten-Muster, LSM, 51, ein einzelnes Detektorsignal 61 erzeugt werden; alternativ können auch nach Aufintegration/Aufsummierung Detektorsignal 61 auf mehreren Licht-und-Schatten-Muster, LSM, 51 basieren.

Beispielsweise kann das Erzeugen S40 der Licht-und-Schatten-Muster, LSM, 51, ein partielles Abschatten und/oder Übersteuern von Licht aus der Umwandlungseinrichtung 40 umfassen. Alternativ oder zusätzlich kann das Erzeugen S50 der Licht-und-Schatten-Muster, LSM, 51, auch das räumlich aufgelöste, gezielte Unterbinden des Umwandelns von Röntgenstrahlung 2 durch die Umwandlungseinrichtung 40 umfassen. Wiederum alternativ oder zusätzlich kann das Erzeugen S50 der Licht-und-Schatten-Muster, LSM, 51, auch das räumlich aufgelöste, gezielte Unterbinden des Detektierens von sichtbarem Licht durch die Detektoreinrichtung 60 umfassen. In jedem dieser Fälle bezieht sich "räumlich aufgelöst" jeweils auf die von dem jeweiligen Licht-und-Schatten-Muster, LSM, 51 vorgegebene Musterfolge von z.B. weißen und schwarzen Pixeln. Jede dieser Varianten hat die Gemeinsamkeit, dass am Ende das von der Detektoreinrichtung 60 erzeugte Detektorsignal 61 nicht nur von dem abgetasteten Objekt 1, sondern auch von dem jeweils angewendeten/erzeugten Licht-und-Schatten-Muster, LSM, 51, abhängig ist.

In einem Schritt S60 wird, basierend auf den Detektorsignalen 61 sowie auf einer vorbestimmten Information über die Licht-und-Schatten-Muster, LSM, 51 ein Abbild 5 des Objekts 1 generiert. Wie im Vorangehenden bereits ausführlich beschrieben wurde, kann dies mittels bekannter Computer-Geisterbildgebungsverfahren ("computational ghost imaging") erfolgen, insbesondere unter Verwendung von vorbestimmten Intensitätsverteilungen I(x,y) für jedes der Licht-und-Schatten-Muster, LSM, 51.

In der vorangegangenen detaillierten Beschreibung sind verschiedene Merkmale zur Verbesserung der Stringenz der Darstellung zusammengefasst worden. Es sollte dabei jedoch klar sein, dass die obige Beschreibung lediglich illustrativer, keinesfalls jedoch beschränkender Natur ist. Sie dient der Abdeckung aller Alternativen, Modifikationen und Äquivalente der verschiedenen Merkmale und Ausführungsbeispiele. Viele andere Beispiele werden dem Fachmann aufgrund seiner fachlichen Kenntnisse in Anbetracht der obigen Beschreibung sofort und unmittelbar klar sein.

Die Ausführungsbeispiele wurden ausgewählt und beschrieben, um die der Erfindung zugrundeliegenden Prinzipien und ihre Anwendungsmöglichkeiten in der Praxis bestmöglich darstellen zu können. Dadurch können Fachleute die Erfindung und ihre verschiedenen Ausführungsbeispiele in Bezug auf den beabsichtigten Einsatzzweck optimal modifizieren und nutzen.

## Patentansprüche

1. Bildgebungsvorrichtung (100) zum Erstellen eines Abbilds (5) eines Objekts (1) durch Bestrahlen des Objekts (1) mit Röntgenstrahlung (2), umfassend:

   einer Röntgenlichtquelle (10) zum Aussenden von Röntgenstrahlung (2);
   einem Aufnahmebereich (30) für ein mit der Röntgenstrahlung (2) abzutastendes Objekt (1);
   eine Umwandlungseinrichtung (40) zum räumlich aufgelösten Umwandeln (S30) von Röntgenstrahlung (2), welche den Aufnahmebereich (30) passiert hat, in sichtbares Licht;
   einer Detektoreinrichtung (60), welche zum Ausgeben eines Detektorsignals (61) basierend auf durch die Detektoreinrichtung (60) erfasstem sichtbaren Licht aus der Umwandlungseinrichtung (40) ausgebildet ist;
   einer Mustergeneratoreinrichtung (50), mittels welcher zeitversetzt eine Vielzahl von Licht-und-Schatten-Mustern, LSM, (51) durch Einwirken auf Röntgenstrahlung (2), welche den Aufnahmebereich (30) passiert hat und/oder auf von der Umwandlungseinrichtung (40) erzeugtes sichtbares Licht derart realisierbar ist, dass jedes Detektorsignal (61) basierend auf einem jeweiligen LSM (51) erzeugt wird; und
   eine Auswerteeinrichtung (60), welche dazu ausgelegt ist, basierend auf den Detektorsignalen (61) sowie auf einer vorbestimmten Information über die LSM (51) das Abbild (5) des Objekts (1) zu generieren.

2. Bildgebungsvorrichtung (100) nach Anspruch 1,
   wobei die Mustergeneratoreinrichtung (50) zwischen der Umwandlungseinrichtung (40) und der Detektoreinrichtung (60) angeordnet ist und die LSM (51) durch partielles Abschatten des sichtbaren Lichts aus der Umwandlungseinrichtung (40) erzeugt.

3. Bildgebungsvorrichtung (100) nach Anspruch 1,

wobei die Mustergeneratoreinrichtung (50) und die Umwandlungseinrichtung (40) derart ineinander integriert sind, dass die Umwandlungseinrichtung (40) nur Röntgenstrahlung (2), welche auf gemäß dem jeweiligen LSM (51) bestimmte Bereiche der Umwandlungseinrichtung (40) auftrifft, in sichtbares Licht umwandelt.

4.  Bildgebungsvorrichtung (100) nach Anspruch 1,
    wobei die Mustergeneratoreinrichtung (50) und die Detektoreinrichtung (60) derart ineinander integriert sind, dass das LSM (51) direkt auf der Detektoreinrichtung (60) erzeugt wird.

5.  Bildgebungsvorrichtung (100) nach einem der Ansprüche 1 bis 4,
    wobei die Detektoreinrichtung (60) eine Auflösung von größer gleich fünf Detektorpixeln und kleiner gleich einhundert Detektorpixeln aufweist.

6.  Bildgebungsvorrichtung (100) nach einem der Ansprüche 1 bis 5,
    wobei mehrere Detektorpixel bei der Auswertung zu einer Ausleseeinheit zusammengefasst werden.

7.  Bildgebungsvorrichtung (100) nach einem der Ansprüche 1 bis 4,
    wobei die Detektoreinrichtung (60) als ein Ein-Detektorpixel-Detektor ausgebildet ist.

8.  Bildgebungsvorrichtung (100) nach einem der Ansprüche 1 bis 7,
    wobei eine Auflösung der LSM (51) größer ist als eine Auflösung der Detektoreinrichtung (60).

9.  Bildgebungsvorrichtung (100) nach einem der Ansprüche 1 bis 8,
    wobei eine Zeitintegration oder Summierung der Detektorsignale (61) in der Detektoreinrichtung (60) durchgeführt wird.

10. Bildgebungsverfahren zum Erstellen eines Abbilds (5) eines Objekts (1) durch Bestrahlen des Objekts (1) mit Röntgenstrahlung (2), umfassend die Schritte:

    Anordnen (S10) eines abzutastenden Objekts (1) in einem Aufnahmebereich (30);
    Aussenden (S20) von Röntgenstrahlung (2) durch eine Röntgenlichtquelle (10) in den Aufnahmebereich (30) zum Beleuchten des abzutastenden Objekts (1);
    räumlich aufgelöstes Umwandeln (S30) von Röntgenstrahlung (2), welche den Aufnahmebereich (30) passiert hat, in sichtbares Licht;
    Erzeugen (S50) von Detektorsignalen (61) basierend auf durch eine Detektoreinrichtung (60) erfasstem sichtbaren Licht;
    Zeitversetztes Erzeugen (S40) einer Vielzahl von Licht-und-Schatten-Mustern, LSM, (51) durch Einwirken auf Röntgenstrahlung (2), welche den Aufnahmebereich (30) passiert hat und/oder auf von der Umwandlungseinrichtung (40) erzeugtes sichtbares Licht derart, dass jedes Detektorsignal (61) basierend auf einem jeweiligen LSM (51) erzeugt wird; und
    Generieren (S60), basierend auf den Detektorsignalen (61) sowie auf einer vorbestimmten Information über die LSM (51), des Abbilds (5) des Objekts (1).

FIG 1

FIG 2

**EUROPÄISCHER RECHERCHENBERICHT**

Nummer der Anmeldung

EP 19 20 1712

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | US 2002/075990 A1 (LANZA RICHARD C [US] ET AL) 20. Juni 2002 (2002-06-20) | 1,8,10 | INV. G01T1/29 |
| Y | * Absätze [0005] - [0007], [0009], [0010], [0017], [0041], [0042], [0043], [0045], [0068], [0099] - [0113]; Abbildungen 1,2A,B,4A-D,8,9,10A,B * | 2-6,9 | |
| Y | US 2009/202043 A1 (CANTU GARY R [US] ET AL) 13. August 2009 (2009-08-13) * Absätze [0033] - [0036], [0043] - [0045]; Abbildungen 1,2,4-8 * | 2-5 | |
| X | EP 2 581 733 A1 (COMMISSARIAT ENERGIE ATOMIQUE [FR]) 17. April 2013 (2013-04-17) * Absätze [0018], [0021], [0024], [0041], [0046], [0086] - [0094]; Abbildungen 3,8A,B,9 * | 1,10 | |
| X | WO 2015/118540 A1 (UNIV BAR ILAN [IL]) 13. August 2015 (2015-08-13) * Seite 2, Zeilen 16-20; Abbildungen 2,3 * * Seite 15, Zeile 14 - Seite 16, Zeile 20 * | 1,10 | RECHERCHIERTE SACHGEBIETE (IPC) G01T |
| X | EP 2 224 850 B1 (UNIV WITWATERSRAND JHB [ZA]) 27. Juni 2012 (2012-06-27) * Absätze [0004], [0017], [0024], [0025], [0052], [0075]; Abbildungen 10,11,18,19 * | 1,7,10 | |
| Y | EP 1 883 839 B1 (QINETIQ LTD [GB]) 25. Juli 2012 (2012-07-25) | 6,9 | |
| A | * Absätze [0007], [0019] - [0020], [0026] - [0027], [0064] - [0065]; Abbildungen 1,2,3,7 * | 1,10 | |

Der vorliegende Recherchenbericht wurde für alle Patentansprüche erstellt

| Recherchenort | Abschlußdatum der Recherche | Prüfer |
|---|---|---|
| Den Haag | 3. April 2020 | Van Ouytsel, Krist'l |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C03)

**ANHANG ZUM EUROPÄISCHEN RECHERCHENBERICHT
ÜBER DIE EUROPÄISCHE PATENTANMELDUNG NR.**

EP 19 20 1712

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

03-04-2020

| Im Recherchenbericht angeführtes Patentdokument | Datum der Veröffentlichung | Mitglied(er) der Patentfamilie | Datum der Veröffentlichung |
|---|---|---|---|
| US 2002075990 A1 | 20-06-2002 | US 2002075990 A1<br>WO 02056055 A2 | 20-06-2002<br>18-07-2002 |
| US 2009202043 A1 | 13-08-2009 | AU 2005295554 A1<br>CA 2584292 A1<br>EP 1810011 A2<br>JP 2008516692 A<br>SG 141445 A1<br>US 2006227933 A1<br>US 2009202043 A1<br>WO 2006044692 A2 | 27-04-2006<br>27-04-2006<br>25-07-2007<br>22-05-2008<br>28-04-2008<br>12-10-2006<br>13-08-2009<br>27-04-2006 |
| EP 2581733 A1 | 17-04-2013 | EP 2581733 A1<br>FR 2981455 A1<br>IL 222341 A<br>US 2013094627 A1 | 17-04-2013<br>19-04-2013<br>30-06-2016<br>18-04-2013 |
| WO 2015118540 A1 | 13-08-2015 | EP 3102976 A1<br>IL 246832 A<br>JP 6605483 B2<br>JP 2017507585 A<br>US 2015381958 A1<br>US 2017163961 A1<br>WO 2015118540 A1 | 14-12-2016<br>31-03-2019<br>13-11-2019<br>16-03-2017<br>31-12-2015<br>08-06-2017<br>13-08-2015 |
| EP 2224850 B1 | 27-06-2012 | EP 2224850 A1<br>JP 5710967 B2<br>JP 2011517336 A<br>US 2011190616 A1<br>WO 2008142543 A1 | 08-09-2010<br>30-04-2015<br>02-06-2011<br>04-08-2011<br>27-11-2008 |
| EP 1883839 B1 | 25-07-2012 | AU 2006250988 A1<br>CA 2608377 A1<br>CN 101228460 A<br>EP 1883839 A1<br>JP 5334574 B2<br>JP 2008542863 A<br>KR 20080021040 A<br>US 2009095912 A1<br>WO 2006125975 A1 | 30-11-2006<br>30-11-2006<br>23-07-2008<br>06-02-2008<br>06-11-2013<br>27-11-2008<br>06-03-2008<br>16-04-2009<br>30-11-2006 |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Nicht-Patentliteratur**

- **ZHANG, HE et al.** Tabletop x-ray ghost imaging with ultra-low radiation. *Optica,* April 2018, vol. 5 (4), 374-377, https://doi.org/10.1364/OPTICA.5.000374